# EUROPEAN PATENT SPECIFICATION

(11) **EP 3 895 544 B1**
(45) Date of publication and mention of the grant of the patent: **28.10.2026**
(21) Application number: 19895274.9
(22) Date of filing: 13.12.2019
(51) Int. Cl.: A23F 3/16, A23L 19/00, A23L 27/00, A23L 2/00, A23L 2/02, A23L 33/105

(54) **METHOD FOR PRODUCING REFINED PRODUCT OF PEAR JUICE**
VERFAHREN ZUR HERSTELLUNG EINES RAFFINIERTEN PRODUKTS AUS BIRNENSAFT
PROCÉDÉ DE PRODUCTION DE PRODUIT RAFFINÉ DE JUS DE POIRE

(30) Priority: 13.12.2018 JP 2018233781; 13.12.2018 JP 2018233782
(43) Date of publication of application: 20.10.2021
(73) Proprietor: Kao Corporation, Chuo-Ku Tokyo 103-8210 (JP)
(72) Inventor: OZEKI, Eri, Haga-gun, Tochigi 321-3497 (JP); HASHIZUME, Kohjiro, Haga-gun, Tochigi 321-3497 (JP); OOKI, Yoriko, Tokyo 131-8501 (JP)
(74) Representative: Vossius & Partner Patentanwälte Rechtsanwälte mbB
(86) International application number: PCT/JP2019/048846
(87) International publication number: WO 2020/122222

(56) References cited:
- JP-A- 2013 188 139
- JP-A- 2018 000 192
- JP-A- 2018 000 192
- JP-A- 2018 174 758
- JP-A- 2018 174 758
- US-A1- 2014 127 370
- KHATABI OMAR ET AL: "Extraction and determination of polyphenols and betalain pigments in the Moroccan Prickly pear fruits (Opuntia ficus indica)", ARABIAN JOURNAL OF CHEMISTRY, ELSEVIER, AMSTERDAM, NL, vol. 9, 15 April 2011 (2011-04-15), XP029786868, ISSN: 1878-5352, DOI: 10.1016/J.ARABJC.2011.04.001
- STÉPHANE VIDAL: "The mouth-feel properties of polysaccharides and anthocyanins in a wine like medium", FOOD CHEMISTRY, vol. 85, no. 4, 1 May 2004 (2004-05-01), NL, pages 519 - 525, XP093165421, ISSN: 0308-8146, DOI: 10.1016/S0308-8146(03)00084-0
- MURAYAMA, H ET AL.: "Effect of storage period on the molecular-mass distribution profile of pectic and hemicellulosic polysaccharides in pears", POSTHARVEST BIOLOGY AND TECHNOLOGY, vol. 40, no. 2, 2006, pages 141 - 148, XP027998459, ISSN: 0925-5214, DOI: 10.1016/j.postharvbio.2006.01.001
- YUKARI MIZUNO, TOMOKO TSUJITANI, YASUNORI HAMAUZU: "Characteristics and Food Function of Cell Wall Components in Some Fruits", ENGEIGAKU KENKYUU, 1 January 2010 (2010-01-01), pages 113 - 120, XP055720100, Retrieved from the Internet <URL:https://www.jstage.jst.go.jp/article/hrj/9/1/9_1_113/_pdf/-char/en> DOI: 10.2503/hrj.9.113

## Description

### Field of the Invention

The present invention relates to a method for producing a refined product of pear juice which has an astringency reducing effect.

### Background of the Invention

Methods for separating a physiologically active substance from a Pyrus plant have been disclosed. Patent Literature 1 states that water was added to a ground product of succulent shoots of pear, the mixture was heated and extracted, the filtrate was concentrated, ethanol was then added, the mixture was centrifuged, and that the obtained supernatant was passed through a column to obtain an arbutin fraction. Patent Literature 2 states that a 70% hydrous ethanol solution was added to freeze-dried leaves of a pear, the mixture was homogenized, filtered to remove insoluble substances, and then concentrated under reduced pressure to obtain an extract and that the extract had a glutathione production promoting action. Patent Literature 3 states that powder of a dried bark or a root of a Pyrus plant was extracted with ethanol, concentrated under reduced pressure, and then separated by cellulose column chromatography and that the obtained fraction was useful for treatment of an immune deficiency syndrome. Patent Literature 4 states that a liquid obtained by fermenting pear juice with lactic acid bacillus, removing insoluble substances by filtration and then adding 1,3-butylene glycol had a retinol-related gene expression promoting action.

On the other hand, one of methods commonly used for extracting polysaccharide ingredients from plants or fungi is ethanol precipitation. Non-Patent Literature 1 states that ethanol was added to fruit juice derived from enzyme-treated Japanese pears or Chinese pears to deposit precipitated polysaccharides, and then the inhibitory activity of an aqueous solution of the polysaccharides on an enzyme such as α-amylase was examined. Non-Patent Literature 2 states that polysaccharides containing pectin and hemicellulose were extracted by repeating a procedure in which ethanol is added to a fruit such as a pear, the mixture is heated, then homogenized, and filtered, an aqueous ethanol solution is added to the residue, and the mixture is heated and filtered to obtain a residue again. Non-Patent Literature 3 states that a polymer substance was precipitated with ethanol from a culture filtrate of pear cells, and arabinogalactan protein was separated from the precipitate.

Catechins are a type of polyphenols, and have various physiological actions including an antioxidant action. However, beverages containing a catechin at a high concentration may involve a feeling of discomfort or a feeling of disgust because of an excessively intense astringency derived from a catechin. Heretofore, studies have been extensively conducted on means for reducing the astringency of a catechin. For example, Patent Literature 5 states that addition of fruit juice of a Rosaceae plant such as a pear reduces the astringency of beverages containing a gallate-type nonpolymeric catechin.

### Citation List

(Patent Literature 1) JP-A-2006-111581
(Patent Literature 2) JP-A-2010-105937
(Patent Literature 3) JP-A-2017-510288
(Patent Literature 4) JP-B-6377879
(Patent Literature 5) JP-A-2018-000192

(Non-Patent Literature 1) Food Research International, 2017, 102: 156-162
(Non-Patent Literature 2) Postharvest Biology and Technology, 2006, 40: 141-148
(Non-Patent Literature 3) Proc. Natl. Acad. Sci, USA, 1994, 91: 10305-10309

### Summary of the Invention

In an embodiment, the present invention provides an astringency reducing agent having a cold ethanol precipitation product of pear juice as an active ingredient.

In another embodiment, the present invention provides an oral composition containing a catechin, which comprises the astringency reducing agent.

In another embodiment, the present description provides a method for producing an astringency reducing agent, comprising:
placing a mixed liquid comprising pear juice and ethanol at a cold temperature to form a precipitate; and
collecting the obtained precipitate, the mixed liquid having an ethanol concentration of from more than 50 v/v% to 70 v/v%.

In still another embodiment, the present invention provides a method for producing a refined product of pear juice, comprising:
placing a mixed liquid comprising pear juice and ethanol at a cold temperature to form a precipitate; and
collecting the obtained precipitate, the mixed liquid having an ethanol concentration of from more than 50 v/v% to 70 v/v%.

### Detailed Description of the Invention

An astringency reducing agent having a higher astringency reducing effect is required. Use of a large amount of a conventional astringency reducing agent containing pear juice or the like (e.g. one described in Patent Literature 5) may produce an off-flavor in a food product containing a catechin, and consequently cause an adverse effect on the flavor of the food product. An astringency reducing agent having little effect on the flavor of a food product is required. The present invention provides a pear juice-derived substance having a reducing effect on the astringency of a catechin and unlikely to cause an off-flavor in a food product, and a method for producing the substance.

The present inventors found that a refined product of pear juice which is an ethanol precipitation product obtained by ethanol precipitation of pear juice under specific conditions has a high redcuing effect on the astringency of a catechin. The present inventors found that the refined product of pear juice is unlikely to produce an off-flavor when added to a food product, and is therefore suitable for reducing astringency from a catechin in a food product containing a catechin.

The method of the present invention enables extraction of an astringency reducing ingredient of pear juice which has been heretofore known as a reducing agent against the astringency of a catechin. Accordingly, the present invention provides a refined product of pear juice which is useful for reduction of astringency. The refined product of pear juice obtained according to the present invention has a more intense astringency reducing effect over pear juice which has been heretofore known as a reducing agent against the astringency of a catechin. Further, the refined product of pear juice is excellent in reducing effect on the astringency of a catechin, and unlikely to produce an off-flavor when added to a food product. Therefore, the refined product of pear juice is capable of reducing an offensive flavor from a catechin in a food product containing a catechin without deterioration of the flavor of the food product.

In an aspect, the present invention provides a method for producing a refined product of pear juice. The refined product of pear juice which is produced according to the present invention is one that is obtained by ethanol precipitation of pear juice under specific conditions.

In the present description, the pear refers to types or classes of pears belonging to Pyrus communis, and examples thereof include bartlett pears, Le Lectier, Claude Blanchet and Cidre pears. The pear is different in type from Japanese pears and Chinese pears (e.g. Pyrus pyrifolia and Pyrus bretshneideri). In the method for producing a refined product of pear juice according to the present invention, the pear juice used as a raw material may be fruit juice from types or classes belonging to Pyrus communis, and may be fruit juice from any one type or class, or a combination of fruit juices from any two or more types or classes.

The pear juice used as a raw material is not particularly limited as long as it is produced from a fruit of a pear by a conventional method. For example, the pear juice may be fruit juice extracted by, for example, pulverizing or squeezing a pear fruit using an apparatus such as a grinder, a mixer, a juice extractor, a grater or a twin-screw extruder, or may be fruit juice obtained by removing peels, seeds and the like from the extracted juice with a strainer or the like. Alternatively, clean fruit juice may be prepared by a method such as centrifugation or filtration. Alternatively, commercially available pear juice may be used. As the pear juice, one produced by the above-described conventional method or a marketed product may be used as such, or may be concentrated or diluted with water and used. For the concentration, it is preferable to employ concentration treatment in which water is removed and other ingredients are maintained, such as boiling concentration, vacuum concentration, freeze concentration or membrane concentration. In the present invention, it is preferable to use concentrated pear juice as raw material pear juice from the viewpoint of improving the yield of a desired refined product of pear juice. For example, the raw material pear juice is preferably pear juice concentrated to a Brix value of about from 60 to 70° Bx.

In the ethanol precipitation of the pear juice, a mixed liquid containing the raw material pear juice mentioned above and ethanol is placed at a cold temperature to form a precipitate, and the obtained precipitate is then collected. The mixed liquid used for the ethanol precipitation also contains water. The water contained in the mixed liquid may include water contained in the raw material pear juice or water of the aqueous ethanol solution, or may be water added separately from the above-mentioned water. As an example, the mixed liquid can be prepared by mixing the raw material pear juice with ethanol and water. As another example, the mixed liquid may be prepared by mixing the raw material pear juice with ethanol or an aqueous ethanol solution and water if necessary. In any of the cases, it is preferable to sufficiently blend the contained ingredients (raw material pear juice, ethanol and water if necessary) by treatment such as agitation, shaking or stirring in preparation of the mixed liquid. The temperature of the liquid during the mixing is not particularly limited, and it is not necessary to heat or cool the liquid. For example, the mixed liquid may be prepared at room temperature.

The concentration of ethanol in the mixed liquid is more than 50 v/v%, preferably 60 v/v% or more for securing the yield of a desired refined product of pear juice. On the other hand, the concentration of ethanol in the mixed liquid is 70 v/v% or less for retaining the astringency reducing effect on the refined product of pear juice and reducing the off-flavor of a food product containing the refined product. Therefore, the concentration of ethanol in the mixed liquid is from more than 50 v/v% to 70 v/v%, more preferably from 60 v/v% to 70 v/v%.

The concentration of pear juice in the mixed liquid is preferably from 5 v/v% to 30 v/v%, more preferably from 10 v/v% to 20 v/v% for securing the yield of a desired refined product of pear juice.

In the present description, the volume% (v/v%) refers to a value calculated on the basis of a volume at 25°C.

The ethanol precipitation of the pear juice is performed at a cold temperature, preferably from -20 to +5°C. For example, the ethanol precipitation at such a cold temperature can be performed by placing the mixed liquid in an atmosphere at a cold temperature (preferably from -20 to +5°C), for example in a refrigerator, or maintaining the mixed liquid at a cold temperature (preferably from -20 to +5°C) with a thermostat or the like. If the temperature during the ethanol precipitation is excessively cold, the mixed liquid may freeze. On the other hand, if the temperature is excessively high, the precipitate formation rate may decrease to the extent that a sufficient yield cannot be secured.

On the other hand, the time for the ethanol precipitation is preferably 1 hour or more, more preferably 24 hours or more for securing the yield of a desired refined product of pear juice. However, if the time is excessively long, an off-flavor of a food product containing the obtained refined product of pear juice may be intensified, and therefore the time for the ethanol precipitation is preferably 7 days or less, more preferably 5 days or less. Therefore, the time for the ethanol precipitation is preferably from 1 hour to 7 days, more preferably from 24 hours to 7 days, even more preferably from 24 hours to 5 days. Alternatively, the time for the ethanol precipitation can be adjusted depending on the temperature condition. For example, with consideration given to the precipitation rate (yield of desired product), the time can be adjusted to be longer at a higher temperature, and adjusted to be shorter at a colder temperature.

Preferred examples of the conditions for ethanol precipitation according to the present invention will be described below. These conditions are illustrative, and do not limit the conditions for ethanol precipitation according to the present invention.

Concentration of ethanol in mixed liquid: from more than 50 v/v% to 70 v/v%, concentration of pear juice in mixed liquid: from 5 v/v% to 30 v/v%, and reaction temperature and time: from -20 to +5°C and from 1 hour to 7 days.

Concentration of ethanol in mixed liquid: from 60 v/v% to 70 v/v%, concentration of pear juice in mixed liquid: from 5 v/v% to 30 v/v%, and reaction temperature and time: from -20 to +5°C and from 24 hours to 7 days.

Concentration of ethanol in mixed liquid: from 60 v/v% to 70 v/v%, concentration of pear juice in mixed liquid: from 5 v/v% to 30 v/v%, and reaction temperature and time: from -20 to +5°C and from 24 hours to 5 days.

Concentration of ethanol in mixed liquid: from 60 v/v% to 70 v/v%, concentration of pear juice in mixed liquid: from 10 v/v% to 20 v/v%, and reaction temperature and time: from -20 to +5°C and from 24 hours to 5 days.

Preferably, the pear juice in the mixed liquid is concentrated pear juice. More preferably, the pear juice in the mixed liquid is concentrated pear juice with from 60 to 70° Bx.

A precipitate is formed in the mixed liquid by the ethanol precipitation. In the present invention, this precipitate is collected from the mixed liquid. For example, the precipitate may be separated from the mixed liquid by filtration, decantation, centrifugation or the like, and collected. The obtained precipitate may be washed with an aqueous ethanol solution if necessary. The collected precipitate can be preserved as such, or used as an astringency reducing agent as described later. Alternatively, the collected precipitate may be prepared in the form of a liquid, a dried product or the like if necessary. For example, a liquid having the collected precipitate dissolved or suspended in ethanol or the like may be prepared, or a dried product may be prepared by a method such as freeze-drying. The obtained liquid or dried product can be used for reducing of astringency described later.

A desired refined product of pear juice can be produced in accordance with the above procedure. The refined product of pear juice has a reducing effect on the astringency of a catechin, and can be used as an astringency reducing agent. The refined product of pear juice is superior in reducing effect on the astringency of a catechin to the raw material pear juice. As compared to the raw material pear juice, the refined product of pear juice less likely produces an off-flavor in a food product (including a beverage) containing the raw material pear juice, and less likely deteriorates the essential flavor of the food product (e.g. a tea-like flavor of a tea beverage containing a catechin). Therefore, the refined product of pear juice obtained according to the present invention can be used for reduction of astringency. The refined product of pear juice is effective preferably for reduction of astringency from a catechin, more preferably for reduction of astringency of a catechin in an oral composition containing a catechin, for example a food product containing a catechin.

The "astringency" in the present description refers to a sense of being numbed in the mouth, which is perceived by shrinkage of the mucous membranes of the entire oral cavity, other than those of taste buds and taste cells. Unlike bitterness perceived by a taste receptor, the astringency is a sense which is not included in the "taste sense". In fact, for the astringency, continuous oral ingestion of an astringent substance accumulates and intensifies the sense of astringency in the oral cavity. On the other hand, for the bitterness, continuous oral ingestion of a bitter substance does not cause such accumulation. Thus, those skilled in the art commonly know that the astringency is a sense entirely different from the bitterness.

The "off-flavor" of a food product in the present description is a flavor impairing a flavor essentially required for the food product, or a flavor different from a flavor essentially required for the food product. For example, a taste or flavor such as sweetness, saltiness, sourness, bitterness, umami, pungency or astringency undesirably added to a food product may eliminate the essential flavor of the food product, or impart an unexpected flavor, resulting in production of an off-flavor in the food product. As a more specific example, when addition of a refined product of pear juice to a tea beverage containing a catechin impairs the tea-like flavor of the beverage, or imparts a flavor different from the tea-like flavor to the beverage, the tea beverage has an "off-flavor".

Examples of the type of catechins whose astringency is reduced by the refined product of pear juice obtained according to the present invention include nonpolymeric catechins, and gallate-type nonpolymeric catechins are preferable. In the present description, the "catechin" is preferably a nonpolymeric catechin, more preferably a gallate-type nonpolymeric catechin. The "nonpolymeric catechin" in the present description is at least one selected from the group consisting of catechin, gallocatechin, epicatechin, epigallocatechin and gallates thereof. The "gallate-type nonpolymeric catechin" in the present description is at least one selected from the group consisting of catechin gallate, gallocatechin gallate, epicatechin gallate and epigallocatechin gallate. These catechins can be extracted from tea leaves of Camellia, or is commercially available.

Accordingly, still another aspect of the present invention relates to providing an astringency reducing agent. The astringency reducing agent of the present invention comprises the above-mentioned refined product of pear juice as an active ingredient. Another aspect of the present invention relates to use of the refined product of pear juice for production of the astringency reducing agent. In an embodiment, the astringency reducing agent can be essentially formed from the refined product of pear juice. In another embodiment, the astringency reducing agent comprises at least the refined product of pear juice.

In a preferred embodiment, the astringency reducing agent is a reducing agent against the astringency of a catechin. In another preferred embodiment, the astringency reducing agent is a reducing agent against the astringency of an oral composition containing a catechin. In a more preferred embodiment, the astringency reducing agent is a reducing agent against the astringency of a catechin in an oral composition containing a catechin. Accordingly, in a preferred embodiment, the astringency reducing agent of the present invention is an oral composition containing a catechin, which comprises the catechin and the refined product of pear juice, and reduces the astringency of a catechin in the oral composition containing a catechin.

Still another aspect of the present invention relates to use of the refined product of pear juice for reduction of astringency. Also disclosed is a method for reducing astringency using the refined product of pear juice. In a preferred embodiment, the reduction of astringency is reduction of the astringency of a catechin. In another preferred embodiment, the reduction of astringency is reduction of the astringency of an oral composition containing a catechin. In a more preferred embodiment, the reduction of astringency is reduction of the astringency of a catechin in an oral composition containing a catechin.

In a preferred embodiment, the method for reducing astringency comprises orally administering the refined product of pear juice to a subject desiring reduction of astringency. Examples of the subject include humans and nonhuman animals, and humans are more preferable. More specific examples of the subject include humans and nonhuman animals orally ingesting a catechin or an oral composition containing the catechin. In an embodiment, the method comprises orally administering a catechin or an oral composition containing the catechin and the refined product of pear juice to the subject. In a preferred embodiment, the method comprises orally administering an oral composition containing a catechin and the refine product of pear juice to the subject. In a preferred embodiment, the method comprises orally administering a catechin and the astringency reducing agent of the present invention to the subject. In a preferred embodiment, the method comprises orally administering an oral composition containing a catechin and the astringency reducing agent to the subject.

In the present description, the amount of the refined product of pear juice used for reducing the astringency of a catechin may be an amount which ensures that a reduction effect on the astringency of the catechin can be obtained, preferably an amount which ensures that a reduction effect on the astringency of the catechin can be obtained, and the flavor of the refined product of pear juice is not perceived, or an offensive off-flavor is not given. In a preferred embodiment, the amount of the refined product of pear juice used for reducing the astringency of a catechin can be determined on the basis of the mass ratio of the refined product (B) of pear juice which is an active ingredient of the present invention to a catechin (A) whose astringency is to be reduced [(B)/(A)]. The mass ratio [(B)/(A)] is preferably 0.0005 or more, more preferably 0.001 or more, even more preferably 0.0025 or more, from the viewpoint of the efficacy of the astringency reducing effect, and preferably 0.8 or less, more preferably 0.1 or less, even more preferably 0.035 or less, from the viewpoint of the flavor balance. Therefore, the mass ratio [(B)/(A)] in the present invention is preferably from 0.0005 to 0.8, more preferably from 0.001 to 0.1, even more preferably from 0.0025 to 0.035. When the mass ratio is in the above-mentioned range, the astringency of a catechin can be reduced without expression of the flavor of the refined product of pear juice.

In the present description, the mass of the refined product of pear juice is a mass in terms of the dried product. In the present description, the content of the refined product of pear juice in terms of the dried product preferably refers to its mass when the refined product of pear juice is a freeze-dried product. However, as described above, the form of the refined product of pear juice for use in the present invention is not limited to a freeze-dried product, and may be a precipitate itself collected after ethanol precipitation, or a liquid prepared from the precipitate.

The astringency reducing agent of the present invention may comprise ingredients other than the refined product of pear juice which is an active ingredient. Examples of the other ingredients include the catechins, and pharmaceutically acceptable carriers, other medicinal ingredients, food product materials and additives acceptable for food products as described later.

In an embodiment, the content of the refined product of pear juice in the astringency reducing agent of the present invention may be 0.0001% by mass or more, and is preferably 0.00015% by mass or more, more preferably 0.00025% by mass or more, even more preferably 0.0005% by mass or more, even more preferably 0.001% by mass or more, even more preferably 0.005% by mass or more, even more preferably 0.01% by mass or more, and the content may be 100% by mass or less, and is preferably 90% by mass or less, more preferably 80% by mass or less, even more preferably 70% by mass or less, with respect to the total amount of the astringency reducing agent. Alternatively, the content of the refined product of pear juice in the astringency reducing agent of the present invention can be from 0.0001 to 100% by mass, from 0.00015 to 100% by mass, from 0.00025 to 100% by mass, from 0.0005 to 100 mass%, from 0.001 to 100 mass%, from 0.005 to 100% by mass, from 0.01 to 100% by mass, from 0.0001 to 90% by mass, from 0.00015 to 90% by mass, from 0.00025 to 90% by mass, from 0.0005 to 90% by mass, from 0.001 to 90% by mass, from 0.005 to 90% by mass, from 0.1 to 90% by mass, from 0.0001 to 80% by mass, from 0.00015 to 80% by mass, from 0.00025 to 80% by mass, from 0.0005 to 80% by mass, from 0.001 to 80% by mass, from 0.005 to 80% by mass, from 0.01 to 80% by mass, from 0.0001 to 70% by mass, from 0.00015 to 70% by mass, from 0.00025 to 70% by mass, from 0.0005 to 70% by mass, from 0.001 to 70% by mass, from 0.005 to 70% by mass or from 0.01 to 70% by mass, and is preferably from 0.001 to 90% by mass, more preferably from 0.005 to 80% by mass, even more preferably from 0.01 to 70% by mass, with respect to the total amount of the astringency reducing agent.

In another embodiment, the content of the refined product of pear juice in the astringency reducing agent of the present invention is preferably 0.0001% by mass or more, more preferably 0.00015% by mass or more, even more preferably 0.00025% by mass or more, and preferably 0.08% by mass or less, more preferably 0.01% by mass or less, even more preferably 0.005% by mass or less, with respect to the total amount of the astringency reducing agent. Alternatively, the content of the refined product of pear juice in the astringency reducing agent of the present invention can be from 0.0001 to 0.08% by mass, from 0.00015 to 0.08% by mass, from 0.00025 to 0.08% by mass, from 0.0001 to 0.01% by mass , from 0.00015 to 0.01% by mass, from 0.00025 to 0.01% by mass, from 0.0001 to 0.05% by mass, from 0.00015 to 0.05% by mass or from 0.00025 to 0.05% by mass, and is preferably from 0.0001 to 0.08% by mass, more preferably from 0.00015 to 0.01% by mass, even more preferably from 0.00025 to 0.005% by mass, with respect to the total amount of the astringency reducing agent.

In an embodiment, the astringency reducing agent of the present invention further comprises a catechin. In this embodiment, the astringency reducing agent of the present invention comprises the catechin and the refined product of pear juice at a ratio of the refined product (B) of pear juice to the catechin (A) [(B)/(A)] as described above.

In the present description, examples of the oral composition containing a catechin, whose astringency is reduced by the refined product of pear juice, include oral pharmaceutical products and quasi-pharmaceutical products, and food products.

Examples of the dosage forms of the pharmaceutical product and quasi-pharmaceutical product include forms capable of being orally administered, for example oral solid preparations such as tablets (including chewable tablets), capsules, granules, powders and troches, and oral liquid preparations such as internally administered solutions and syrups. Preparations with these dosage forms can be prepared in accordance with a conventional method by appropriately combining the ethanol precipitation product of pear juice with pharmaceutically acceptable carriers (e.g. an excipient, a binder, an extender, a disintegrant, a surfactant, a lubricant, a dispersant, a buffering agent, a preservative, a flavoring agent, an aroma, a coating and a diluent), other medicinal ingredients and the like.

Preferably, the oral composition containing a catechin is a food product containing a catechin. The form of the food product can be solid, semisolid or liquid (e.g. a beverage). Therefore, the food product can be rephrased as a food or drink product. The food product containing a catechin includes food products for humans and food products for nonhuman animals (e.g. feeds, pet foods and beverages for pets). Alternatively, the food product may be a supplement for humans or nonhuman animals which has the form of a tablet, a capsule, a granule, a powder, a solution, a syrup or the like.

The food product containing a catechin is preferably a beverage containing a catechin. The beverage may be a tea beverage or a non-tea beverage. Examples of the tea beverage include green tea beverages, oolong tea beverages and black tea beverages. Examples of the non-tea beverage include non-alcoholic beverages such as carbonated beverages, fruit juice, vegetable juice, sports drinks, isotonic drinks, enhanced water, bottled water, soft drinks with minute amounts of flavoring, coffee beverages, energy drinks and beauty health drinks, and alcoholic drinks such as beer, wine, rice wine, plum liquor, sparkling liquor, whisky, brandy, clear liquor, rum, gin and liqueurs. The form of the beverage is not particularly limited, and may be liquid, gelatinous or slurry as long as it is easily ingested.

The food product containing a catechin can be prepared in accordance with a conventional method by appropriately combining the refined product of pear juice with any food product material or other active ingredients, or additives acceptable for food products (e.g. a solvent, a softening agent, an oil, an emulsifier, an antiseptic agent, an acidulant, a sweetening agent, a bittering agent, a pH adjuster, a stabilizer, a colorant, an ultraviolet absorber, an antioxidant, a moisturizing agent, a thickener, a sticking agent, a dispersant, a fluidity improving agent, a wetting agent, an aroma, a seasoning agent and a seasoning modifier).

The content of the catechin in the oral composition containing a catechin may be from 0.001 to 0.8% by mass with respect to the total amount of the composition. From the viewpoint of the physiological effect of the catechin, the content of the catechin is preferably 0.005% by mass or more, more preferably 0.008% by mass or more, even more preferably 0.03% by mass or more, with respect to the total amount of the composition. On the other hand, for preventing the composition from having an excessively astringent taste, the content of the catechin is preferably 0.7% by mass or less, more preferably 0.6% by mass or less, even more preferably 0.4% by mass or less, with respect to the total amount of the composition. Therefore, the content of the catechin in the oral composition containing a catechin is preferably from 0.005 to 0.7% by mass, more preferably from 0.008 to 0.6% by mass, even more preferably from 0.03 to 0.4% by mass, with respect to the total amount of the composition. The content of the catechin in the above-described oral composition containing a catechin is the total amount of catechins contained in the composition, preferably the total amount of the above-described nonpolymeric catechins contained in the composition, more preferably the total amount of the above-described gallate-type nonpolymeric catechins contained in the composition.

The content of the refined product of pear juice in the oral composition containing a catechin can be appropriately set according to a type of the composition and a desired astringent intensity. The content of the refined product of pear juice in the composition is preferably 0.0001% by mass or more, more preferably 0.00015% by mass or more, even more preferably 0.00025% by mass or more, with respect to the total amount of the composition, from the viewpoint of the astringency reducing effect, and is preferably 0.08% by mass or less, more preferably 0.01% by mass or less, even more preferably 0.005% by mass or less, with respect to the total amount of the composition, from the viewpoint of reducing the flavor of the refined product of pear juice. Alternatively, the content of the refined product of pear juice in the composition is preferably from 0.0001 to 0.08% by mass, more preferably from 0.00015 to 0.01% by mass, even more preferably from 0.00025 to 0.005% by mass, with respect to the total amount of the composition. Preferably, the value of the mass ratio of the refined product (B) of pear juice to the catechin (A) [(B)/(A)] in the oral composition containing a catechin is as described above. Therefore, the content of the refined product of pear juice in the composition can be adjusted on the basis of the content of the catechin in the composition so that the mass ratio [(B)/(A)] can be achieved.

The content of the refined product of pear juice in the food product containing a catechin may be an amount effective for reducing the astringency of the catechin, preferably an amount which ensures that reducing effect on the astringency of the catechin is achieved, and that the flavor of the refined product of fruit juice is not perceived, or an offensive off-flavor is not given. The content of the refined product of pear juice in the food product containing a catechin can be appropriately set according to a type of the food product and an astringent intensity. As a preferred example, the content of the refined product of pear juice (in terms of the dried product) in the food product containing a catechin is from 2.5 to 50 ppm by mass with respect to the total amount of the food product, or from 0.25 to 5% by mass per 100% by mass of the catechin with respect to the total amount of the food product. In the food product, the content of the refined product of pear juice in terms of the dried product preferably refers to its mass when the refined product of pear juice is a freeze-dried product. However, as described above, the form of the refined product of pear juice added to the food product containing a catechin is not limited to a freeze-dried product, and may be a precipitate itself collected after ethanol precipitation, or a liquid prepared from the precipitate.

### Examples

### Reference Example 1: Measurement of astringent intensity

Ten standard aqueous solutions having different astringent intensities were prepared by adjusting the concentrations of a catechin preparation (Teavigo manufactured by DSM Nutrition Company; containing epigallocatechin gallate at 94% by mass or more) added stepwise as in Table **1.** On the basis of sensory evaluation, four dedicated panelists each selected a standard aqueous solution perceived to be comparable in astringent intensity to a test beverage from the ten standard aqueous solutions. The astringent intensities of the selected standard aqueous solutions were averaged among the panelists, and the average was defined as a value of the astringent intensity of the test beverage.

**[Table 1]**

| Standard aqueous solution | |
|---|---|
| Astringent intensity | Concentration of catechin preparation (g/100 mL aq.) |
| 1 | 0.01 |
| 2 | 0.02 |
| 3 | 0.03 |
| 4 | 0.04 |
| 5 | 0.05 |
| 6 | 0.06 |
| 7 | 0.07 |
| 8 | 0.08 |
| 9 | 0.09 |
| 10 | 0.10 |

### Example 1: Preparation of refined product of pear juice (Pear juice)

As the pear juice, pear juice concentrated by a factor of 6 (obtained from KAKOH CO., LTD.; Brix 67.0; product obtained by treating extracted juice of a fruit with pectinase and performing filtration, followed by concentrating the filtrate under reduced pressure) was used.

### (Production Example 1: Production of 60% ethanol precipitate from pear juice)

90 mL of ion-exchanged water and 180 mL of ethanol at 99.5 v/v% were added to 30 mL of pear juice concentrated by a factor of 6, and the mixture was shaken. The obtained mixed liquid was left standing in a refrigerator at -20°C for 4 days, and the precipitate was then separated by centrifugation. The obtained precipitate was washed with 70 v/v% ethanol, and then dissolved in ion-exchanged water, and the solution was freeze-dried. The yield of the precipitate was 26.5 mg, and the percentage yield of the precipitate was 0.066% by mass.

### (Production Example 2: Production of 65% ethanol precipitate from pear juice)

75 mL of ion-exchanged water and 195 mL of ethanol at 99.5 v/v% were added to 30 mL of pear juice concentrated by a factor of 6, and the mixture was shaken. The obtained mixed liquid was left standing in a refrigerator at -20°C for 4 days, and the precipitate was then separated by centrifugation. The obtained precipitate was washed with 70 v/v% ethanol, and then dissolved in ion-exchanged water, and the solution was freeze-dried. The yield of the precipitate was 74.8 mg, and the percentage yield of the precipitate was 0.19% by mass.

### (Production Example 3: Production of 70% ethanol precipitate from pear juice-1)

30 mL of ion-exchanged water and 140 mL of ethanol at 99.5 v/v% were added to 30 mL of pear juice concentrated by a factor of 6, and the mixture was shaken. The obtained mixed liquid was left standing in a refrigerator at 4°C for 4 days, and the supernatant was then removed by decantation to obtain a precipitate. The obtained precipitate was washed with 70 v/v% ethanol, and then dissolved in ion-exchanged water, and the solution was freeze-dried. The yield of the precipitate was 0.959 g, and the percentage yield of the precipitate was 2.4% by mass.

### (Production Example 4: Production of 70% ethanol precipitate from pear juice-2)

30 mL of ion-exchanged water and 140 mL of ethanol at 99.5 v/v% were added to 30 mL of pear juice concentrated by a factor of 6, and the mixture was shaken. The obtained mixed liquid was left standing in a refrigerator at -20°C for 4 days, and the supernatant was then removed by decantation to obtain a precipitate. The obtained precipitate was washed with 70 v/v% ethanol, and then dissolved in ion-exchanged water, and the solution was freeze-dried. The yield of the precipitate was 2.75 g, and the percentage yield of the precipitate was 6.9% by mass.

### (Production Example 5: Production of 75% ethanol precipitate from pear juice)

20 mL of ion-exchanged water and 150 mL of ethanol at 99.5 v/v% were added to 30 mL of pear juice concentrated by a factor of 6, and the mixture was shaken. The obtained mixed liquid was left standing in a refrigerator at 4°C for 4 days, and the supernatant was then removed by decantation to obtain a precipitate. The obtained precipitate was washed with 80 v/v% ethanol, and then dissolved in ion-exchanged water, and the solution was freeze-dried. The yield of the precipitate was 3.65 g, and the percentage yield of the precipitate was 9.1% by mass.

### (Comparative Example 1: Production of 50% ethanol precipitate from pear juice)

120 mL of ion-exchanged water and 150 mL of ethanol at 99.5 v/v% were added to 30 mL of pear juice concentrated by a factor of 6, and the mixture was shaken. The obtained mixed liquid was left standing in a refrigerator at -20°C for 5 days. However, a precipitate was not formed.

Table 2 shows the conditions for production of the precipitates obtained in Production Examples 1 to 5 and Comparative Example 1.

**[Table 2]**

| Refined product of pear juice | Comparative Example 1 | Production Example 1 | Production Example 2 | Production Example 3 | Production Example 4 | Production Example 5 |
|---|---|---|---|---|---|---|
| Concentration in mixed liquid (v/v%) | | | | | | |
| Pear juice | 10 | 10 | 10 | 15 | 15 | 15 |
| Ethanol | 50 | 60 | 65 | 70 | 70 | 75 |

| Standing | | | | | | |
|---|---|---|---|---|---|---|
| Temperature | -20°C | -20°C | -20°C | +4°C | -20°C | +4°C |
| Time | 5 days | 4 days | 4 days | 4 days | 4 days | 4 days |
| Percentage yield (% by mass) | - | 0.066 | 0.19 | 2.4 | 6.9 | 9.1 |

### Example 2: Reducing effect on astringency of refined product of pear juice

### (Preparation of test beverage)

A catechin preparation (Teavigo manufactured by DSM Nutrition Company; containing epigallocatechin gallate at 94% by mass or more) at 0.10% by mass and each of the precipitates obtained in Production Examples 1 to 5 were dissolved by 2.5 ppm by mass in ion-exchanged water to prepare test beverages 1 to 5 as in Table 3.

### (Measurement of astringent intensity)

The astringent intensities of the test beverages 1 to 5 were measured in accordance with the procedure of Reference Example 1. Table 3 shows the results. Beverages containing precipitates with ethanol at 70% or less in Production Examples 1 to 4 (test beverages 1 to 4) had an excellent astringency reducing effect.

**[Table 3]**

| Test beverage No. | 1 | 2 | 3 | 4 | 5 |
|---|---|---|---|---|---|
| Concentration of catechin preparation (% by mass) | 0.10 | 0.10 | 0.10 | 0.10 | 0.10 |
| Precipitate from pear juice | Production Example 1 | Production Example 2 | Production Example 3 | Production Example 4 | Production Example 5 |
| (concentration: ppm by mass) | (2.5) | (2.5) | (2.5) | (2.5) | (2.5) |
| Astringent intensity | 7.6 | 7.8 | 7.3 | 7.0 | 9.5 |

### Example 3: Evaluation of flavor of beverage containing refined product of pear juice

### (Preparation of test beverage)

Each of the precipitates obtained in Production Examples 1 to 5 was dissolved by 25 ppm by mass in a green tea beverage (Healthya Green Tea a: Kao Corporation; containing 540 mg of a catechin per 350 mL) to prepare test beverages 1 to 5. The pear juice concentrated by a factor of 6, which had been used in Example 1, was dissolved by 800 ppm by mass in the same green tea beverage (Healthya Green Tea a) to prepare a comparative beverage 1.

### (Flavor evaluation)

For the test beverages 1 to 5 and the comparative beverage 1, four dedicated panelists sensorily evaluated the astringency and the off-flavor in accordance with the following evaluation criteria.

### (Astringency)

The panelists determined whether or not astringency was reduced as compared with Healthya Green Tea a. The test beverage was rated A when the number of panelists who determined that astringency was reduced was 3 or 4, the test beverage was rated B when the number of such panelists was 2, and the test beverage was rated C when the number of such panelists was 0 or 1.
A: Astringency is reduced.
B: Astringency is slightly reduced.
C: Astringency is not reduced.

### (Off-flavor)

The panelists determined whether or not an off-flavor was sensed (a decrease in tea-like flavor) as compared with Healthya Green Tea a. The test beverage was rated A when the number of panelists who determined that an off-flavor was sensed was 3 or 4, the test beverage was rated B when the number of such panelists was 2, and the test beverage was rated C when the number of such panelists was 0 or 1.
A: An off-flavor is not sensed.
B: An off-flavor is slightly sensed.
C: An off-flavor is sensed.

Table 4 shows the results. The tea beverages containing the precipitates with ethanol at 70% or less in Production Examples 1 to 4 (test beverages 1 to 4) had less astringency, less off-flavor and hence a better tea-like flavor over the tea beverage containing raw material pear juice (comparative beverage 1) and the tea beverage containing the precipitates with ethanol at 75% or less in Production Example 5 (test beverage 5).

**[Table 4]**

| Test beverage No. | 1 | 2 | 3 | 4 | 5 | Comparative beverage 1 |
|---|---|---|---|---|---|---|
| Precipitate from pear juice (concentration: ppm by mass) | Production Example 1 | Production Example 2 | Production Example 3 | Production Example 4 | Production Example 5 | Concentrated pear juice |
| | (25) | (25) | (25) | (25) | (25) | (800) |
| Astringency | A | A | A | A | C | B |
| Off-flavor | A | A | A | B | C | C |

## Claims

1. Use of a product of ethanol precipitation at cold temperature from pear juice for reducing of astringency, wherein the ethanol precipitation is ethanol precipitation in ethanol at from more than 50 v/v% to 70 v/v%.

2. The use according to claim 1, wherein the astringency is the astringency of a catechin.

3. The use according to claim 2, wherein the astringency is the astringency of an oral composition containing a catechin.

4. The use according to claim 3, wherein the oral composition containing a catechin is a beverage containing a catechin.

5. The use according to any one of claims 1 to 4, wherein the ethanol precipitation is ethanol precipitation in ethanol at from 60 v/v% to 70 v/v%.

6. The use according to any one of claims 1 to 5, wherein the cold temperature is from -20 to +5°C.

7. A method for producing a refined product of pear juice, comprising:
placing a mixed liquid comprising pear juice and ethanol at a cold temperature to form a precipitate; and
collecting the obtained precipitate, wherein
the ethanol concentration of the mixed liquid is from more than 50 v/v% to 70 v/v%.

8. The method according to claim 7, wherein the ethanol concentration of the mixed liquid is from 60 v/v% to 70 v/v%.

9. The method according to claim 7 or 8, wherein the cold temperature is from -20 to +5°C.

10. The method according to any one of claims 7 to 9, wherein the time for the placement at the cold temperature is from 1 hour to 7 days.

11. The method according to any one of claims 7 to 10, wherein the concentration of pear juice in the mixed liquid is from 5 v/v% to 30 v/v%.

12. The use according to any one of claims 1 to 6, wherein a product of ethanol precipitation at cold temperature from pear juice is a refined product of pear juice produced by the method according to any one of claims 7 to 11.

13. An astringency reducing agent having as an active ingredient a refined product of pear juice produced by the method according to any one of claims 7 to 11.

14. An oral composition containing a catechin, comprising the astringency reducing agent according to claim 13.

15. The oral composition containing a catechin according to claim 14, which is a beverage.

## Patentansprüche

1. Verwendung eines durch Ethanolfällung bei niedriger Temperatur aus Birnensaft gewonnenen Produkts zur Verringerung der Adstringenz, wobei die Ethanolfällung eine Ethanolfällung in Ethanol von mehr als 50 Vol.-% bis 70 Vol.-% ist.

2. Die Verwendung gemäß Anspruch 1, wobei es sich bei der Adstringenz um die Adstringenz eines Catechins handelt.

3. Die Verwendung gemäß Anspruch 2, wobei es sich bei der Adstringenz um die Adstringenz einer ein Catechin enthaltenden oralen Zusammensetzung handelt.

4. Die Verwendung gemäß Anspruch 3, wobei es sich bei der Catechin enthaltenden oralen Zusammensetzung um ein Catechin enthaltendes Getränk handelt.

5. Die Verwendung gemäß einem der Ansprüche 1 bis 4, wobei die Ethanolfällung eine Ethanolfällung in Ethanol von 60 Vol.-% bis 70 Vol.-% ist.

6. Die Verwendung gemäß einem der Ansprüche 1 bis 5, wobei die niedrige Temperatur bei -20 bis +5°C liegt.

7. Das Verfahren zur Herstellung eines raffinierten Produkts aus Birnensaft, umfassend:
Halten eines Flüssigkeitsgemischs umfassend Birnensaft und Ethanol bei niedriger Temperatur, um einen Niederschlag zu bilden; und
Gewinnen des erhaltenen Niederschlags, wobei
die Ethanolkonzentration des Flüssigkeitsgemischs mehr als 50 Vol.-% bis 70 Vol.-% beträgt.

8. Das Verfahren gemäß Anspruch 7, wobei die Ethanolkonzentration des Flüssigkeitsgemischs 60 Vol.-% bis 70 Vol.-% beträgt.

9. Das Verfahren gemäß Anspruch 7 oder 8, wobei die niedrige Temperatur bei -20 bis +5°C liegt.

10. Das Verfahren gemäß einem der Ansprüche 7 bis 9, wobei die Dauer des Haltens bei niedriger Temperatur 1 Stunde bis 7 Tage beträgt.

11. Das Verfahren gemäß einem der Ansprüche 7 bis 10, wobei die Konzentration an Birnensaft in dem Flüssigkeitsgemisch von 5 Vol.-% bis 30 Vol.-% beträgt.

12. Die Verwendung gemäß einem der Ansprüche 1 bis 6, wobei ein durch Ethanolfällung bei niedriger Temperatur aus Birnensaft gewonnenes Produkt ein raffiniertes Produkt aus Birnensaft ist, welches durch das Verfahren gemäß einem der Ansprüche 7 bis 11 hergestellt wird.

13. Ein Mittel zur Verringerung der Adstringenz, welches als Wirkstoff ein raffiniertes Produkt aus Birnensaft enthält, welches durch das Verfahren gemäß einem der Ansprüche 7 bis 11 hergestellt wird.

14. Eine Catechin enthaltende orale Zusammensetzung, welche das Mittel zur Verringerung der Adstringenz gemäß Anspruch 13 umfasst.

15. Die Catechin enthaltende orale Zusammensetzung gemäß Anspruch 14, bei der es sich um ein Getränk handelt.

## Revendications

1. Utilisation d'un produit de précipitation d'éthanol à température froide provenant d'un jus de poire pour réduire l'astringence, dans laquelle la précipitation d'éthanol est une précipitation d'éthanol dans de l'éthanol à plus de 50 % en v/v jusqu'à 70 % en v/v.

2. Utilisation selon la revendication 1, dans laquelle l'astringence est l'astringence d'une catéchine.

3. Utilisation selon la revendication 2, dans laquelle l'astringence est l'astringence d'une composition orale contenant une catéchine.

4. Utilisation selon la revendication 3, dans laquelle la composition orale contenant une catéchine est une boisson contenant une catéchine.

5. Utilisation selon l'une quelconque des revendications 1 à 4, dans laquelle la précipitation d'éthanol est une précipitation d'éthanol dans de l'éthanol à 60 % en v/v jusqu'à 70 % en v/v.

6. Utilisation selon l'une quelconque des revendications 1 à 5, dans laquelle la température froide va de -20 à +5°C.

7. Méthode de production d'un produit rafiné de jus de poire, comprenant :
le placement d'un liquide mélangé comprenant du jus de poire et de l'éthanol à une température froide pour former un précipité ; et
la collecte du précipité obtenu, où
la concentration en éthanol du liquide mélangé va de plus de 50 % en v/v à 70 % en v/v.

8. Méthode selon la revendication 7, dans laquelle la concentration en éthanol du liquide mélangé va de 60 % en v/v à 70 % en v/v.

9. Méthode selon la revendication 7 ou 8, dans laquelle la température froide va de -20 à +5°C.

10. Méthode selon l'une quelconque des revendications 7 à 9, dans laquelle le temps pour le placement à la température froide va de 1 heure à 7 jours.

11. Méthode selon l'une quelconque des revendications 7 à 10, dans laquelle la concentration en jus de poire dans le liquide mélangé va de 5 % en v/v à 30 % en v/v.

12. Utilisation selon l'une quelconque des revendications 1 à 6, dans laquelle un produit de précipitation d'éthanol à température froide provenant de jus de poire est un produit raffiné de jus de poire produit par la méthode selon l'une quelconque des revendications 7 à 11.

13. Agent réducteur d'astringence ayant en tant qu'ingrédient actif un produit raffiné de jus de poire produit par la méthode selon l'une quelconque des revendications 7 à 11.

14. Composition orale contenant une catéchine, comprenant l'agent réducteur d'astringence selon la revendication 13.

15. Composition orale contenant une catéchine selon la revendication 14, qui est une boisson.
